# EUROPEAN PATENT APPLICATION

(11) **EP 2 559 452 A1**
(43) Date of publication of application: **20.02.2013**
(21) Application number: 12177410.3
(22) Date of filing: 23.07.2012
(51) Int. Cl.: A61M 25/09

(54) **Guidewire**

(30) Priority: 19.08.2011 JP 2011179466
(71) Applicant: ASAHI INTECC CO., LTD., Nagoya-shi, Aichi 463-0024 (JP)
(72) Inventor: Tsunezumi, Atushi c/o ASAHI INTECC CO., LTD., Nagoya-shi, Aichi 463-0024 (JP); Namima, Satoshi c/o ASAHI INTECC CO., LTD., Nagoya-shi, Aichi 463-0024 (JP)
(74) Representative: Angermeier, Simon Andreas

(57) **Abstract**

A guidewire (1) includes a core shaft (2), a hollow coil body (3, 3a), and a hollow resin body (4, 20, 30). The core shaft (2) includes a distal portion (2a), a proximal portion (2b), and a center portion (2c) that is sandwiched between the distal portion (2a) and the proximal portion (2b). The hollow coil body (3, 3a) covers the distal portion (2a) when the distal portion (2a) is housed in the hollow coil body (3, 3a). The hollow resin body (4, 20, 30) covers the center portion (2c) when the center portion (2c) is housed in the hollow resin body (4, 20, 30). The center portion (2c) has a tapered shape such that the diameter of the center portion (2c) decreases from a proximal end portion side of the center portion (2c) toward a distal end portion side of the center portion (2c), and a rigidity of the hollow resin body (4, 20, 30) is higher than a rigidity of the hollow coil body (3, 3a).

## Description

### BACKGROUND OF THE INVENTION

### 1. Field of the Invention

The present invention relates to a guidewire.

### 2. Description of the Related Art

A known example of medical devices that have been used in percutaneous transluminal coronary angioplasty is a guidewire used for guiding a device, such as a balloon or a stent, to a lesion.

As an example of such a guidewire, Japanese Unexamined Patent Application Publication No. 03-90166 discloses a guidewire that includes a hollow metallic hypotube positioned in a proximal portion of the guidewire, a resin tube that is connected to the hypotube and has multiple holes, a coil body that is connected to the resin tube and positioned in a distal portion of the guidewire, and a core shaft that penetrates through the inside of the metallic hypotube, the resin tube, and the coil body from the proximal side to the distal side of the guidewire. In this guidewire, the distal side of the core shaft coincides with the distal side of the guidewire, while the proximal side of the core shaft coincides with the proximal side of the guidewire. The distal portion of the guidewire is inserted into a human body and reaches a lesion while the proximal portion of the guidewire is operated by an operator such as a doctor.

The guidewire described in Japanese Unexamined Patent Application Publication No. 03-90166 is described as having a highly flexible distal portion.

In the case, however, where the guidewire according to Japanese Unexamined Patent Application Publication No. 03-90166 is used to transport a device such as a stent to a lesion, the guidewire is more likely to meander and deviate from a desired route at a sharply curved portion of a blood vessel (also simply referred to as a curved portion, below) due to the transporting of the device. Thus, the guidewire has a low efficiency in terms of delivering devices (referred to as a device delivering efficiency, below). This problem becomes particularly marked when a guidewire has a small diameter for use in treatment of a lesion at a peripheral blood vessel having a smaller diameter because the guidewire has a low rigidity.

### SUMMARY OF THE INVENTION

Therefore, it is the object of the invention to provide a guidewire having a high device delivering efficiency.

This object is achieved by a guidewire according to claim 1. Preferred embodiments of the invention are subject matter of dependent claims.

The inventors have studied the cause of the above problem from various viewpoints. Consequently, the inventors have found that a portion of an existing guidewire that is constituted by the resin tube and a corresponding part of the core shaft penetrating through the inside of the tube (this part is also referred to as a tube region, below) is bent at an almost uniform curvature, and does not have a structure in which the rigidity gradually decreases toward a tip end portion (the structure is also referred to as a gradually-changing structure). The inventors have thus concluded that the reason why the guidewire easily meanders due to the transporting of the device when the guidewire is pushed into a blood vessel and then a tube region of the guidewire arrives at a curved portion is because the tube region cannot sufficiently follow the curve of the blood vessel.

On the basis of these findings, the inventors have performed further studies to improve the device delivering efficiency of a guidewire, and have finally attained a guidewire according to the present invention in the following manner. Specifically, the inventors have found that the tube region can have a desirable gradually-changing structure and thus the guidewire can exert a desirable device delivering efficiency during use if a part of a core shaft constituting the tube region is formed in a tapered shape, rather than in a columnar (cylindrical) shape having a uniform diameter. In addition to this finding, the flexibility of the distal portion of the guidewire is maintained, so that the guidewire according to the present invention is obtained.

The guidewire according to the present invention is a guidewire that includes a core shaft, a hollow coil body, and a hollow resin body. The core shaft includes a distal portion, a proximal portion, and a center portion that is disposed between the distal portion and the proximal portion. The hollow coil body covers the distal portion when the distal portion is housed in the hollow coil body. The hollow resin body covers the center portion when the center portion is housed in the hollow resin body. The center portion has a tapered shape such that the diameter of the center portion decreases from a proximal end portion side of the center portion toward a distal end portion side of the center portion, and a rigidity of the hollow resin body is higher than a rigidity of the hollow coil body.

Referring now to the drawings, the details of a guidewire according to an embodiment of the present invention will be described in terms of structures and effects.

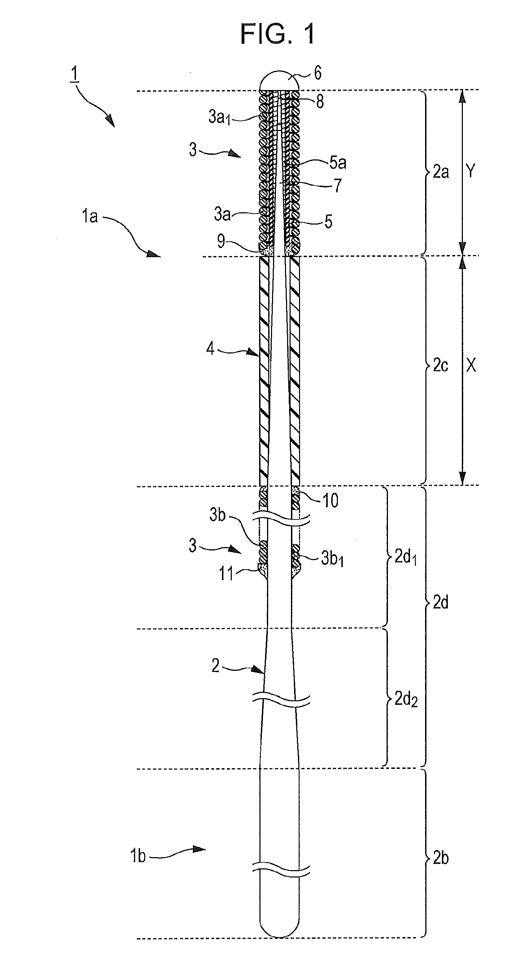

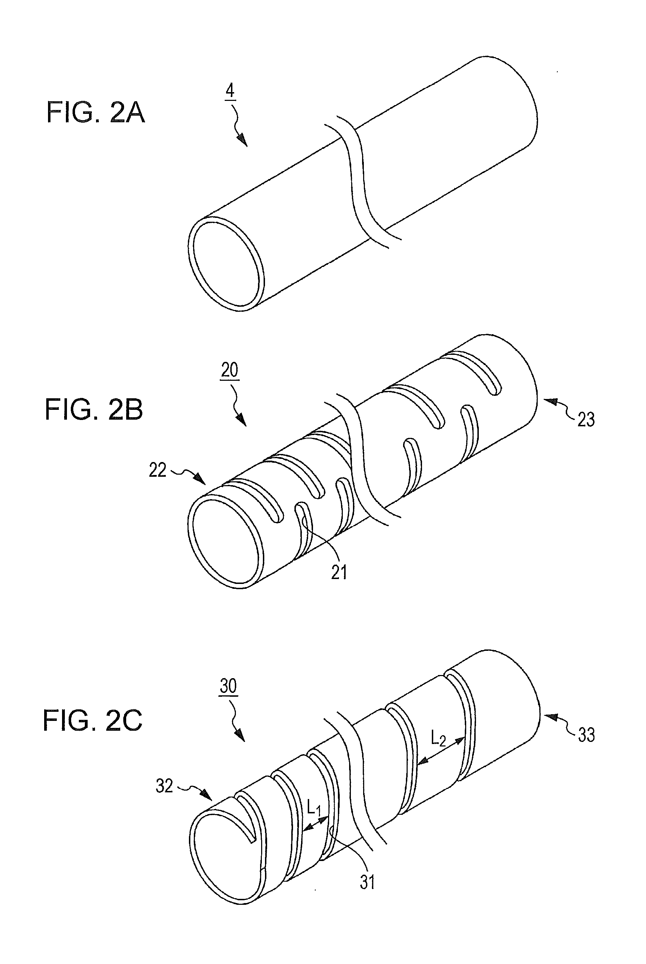

Fig. 1 schematically illustrates a guidewire according to a first embodiment of the present invention when viewed in plan. Fig. 2A schematically illustrates a hollow resin body of the guidewire illustrated in Fig. 1 when viewed in perspective.

The guidewire 1 according to the first embodiment illustrated in Fig. 1 includes a core shaft 2, hollow coil bodies 3, and a hollow resin body 4.

The core shaft 2 includes a distal portion 2a, a proximal portion 2b, and a center portion 2c that is sandwiched between the distal portion 2a and the proximal portion 2b. As illustrated in Fig. 1, a cylindrical first joint portion 2d₁, which is connected to the center portion 2c, and a tapered second joint portion 2d₂, which is connected to the proximal portion 2b, constitute a joint portion 2d that is disposed between the center portion 2c and the proximal portion 2b. The shape of the joint portion 2d is not particularly limited, and may be a shape such as a cylindrical or tapered shape, or the above-described combination of cylindrical and tapered shapes. Alternatively, the joint portion 2d may be omitted and the center portion 2c and the proximal portion 2b may be directly connected.

The center portion 2c of the core shaft 2 has a tapered shape such that the diameter of the center portion 2c decreases from a proximal end portion (first joint portion 2d₁) side to a distal end portion (distal portion 2a) side.

Since the center portion 2c located between the distal portion 2a, which is most flexible, and the proximal portion 2b, which is most rigid, has a tapered shape, the rigidity of the center portion 2c gradually decreases from the proximal portion 2b side to the distal portion 2a side.

One of the hollow coil bodies 3 covers the distal portion 2a when the distal portion 2a is housed in the hollow coil body 3.

The hollow resin body 4 illustrated in Figs. 1 and 2A covers the center portion 2c when the center portion 2c is housed in the hollow resin body 4. The rigidity of the hollow resin body 4 is higher than the rigidity of the hollow coil bodies 3.

The rigidity of the distal-side portion 1a of the guidewire 1 gradually decreases from a proximal end portion side of a tube region X, which is constituted by the center portion 2c and the hollow resin body 4 covering the center portion 2c, toward a distal end portion side of the tube region X. The rigidity of a distal region Y, which is constituted by the distal portion 2a and one of the hollow coil bodies 3 covering the distal portion 2a, is lower than that of the distal end portion side of the tube region X. Consequently, the flexibility of the distal-side portion 1a of the guidewire 1 gradually increases toward the tip.

Compared to an existing guidewire in which not only a distal portion but also a center portion are covered by a hollow coil body, the guidewire 1 according to the embodiment of the present invention has such a degree of rigidity that the tube region X can maintain its device delivering efficiency since the center portion 2c is covered by the hollow resin body 4 having a higher rigidity than the hollow coil bodies 3.

Thus, even when the guidewire 1 according to the embodiment of the present invention is pushed into a blood vessel and then the tube region X reaches a curved portion, the distal-side portion 1a constituted by the tube region X and the distal region Y is capable of following the curve of the blood vessel. Moreover, the guidewire 1 is less likely to meander while delivering a device since the tube region X has a certain degree of rigidity. Accordingly, the guidewire 1 according to the embodiment of the present invention is excellent in terms of device delivering efficiency.

### BRIEF DESCRIPTION OF THE DRAWINGS

Fig. 1 schematically illustrates a guidewire according to a first embodiment of the present invention when viewed in plan.
Fig. 2A schematically illustrates a hollow resin body of the guidewire illustrated in Fig. 1 when viewed in perspective.
Fig. 2B schematically illustrates a hollow resin body of a guidewire according to a third embodiment when viewed in perspective.
Fig. 2C schematically illustrates a hollow resin body of a guidewire according to a fourth embodiment when viewed in perspective.

### DESCRIPTION OF THE PREFERRED EMBODIMENTS

### First Embodiment

Referring to the drawings, a guidewire 1 according to a first embodiment of the present invention will be described below.

The guidewire 1 according to the first embodiment illustrated in Fig. 1 includes a core shaft 2, hollow coil bodies 3 including a distal hollow coil body 3a and a proximal hollow coil body 3b, a hollow resin body 4, and a hollow stranded body 5.

The core shaft 2 has portions in order from the distal side to the proximal side, i.e., a distal portion 2a, a center portion 2c, a first joint portion 2d₁, a second joint portion 2d₂, and a proximal portion 2b. The distal portion 2a has the smallest diameter and the lowest rigidity and thus is most flexible among the portions, and the proximal portion 2b has the largest diameter and the highest rigidity and thus is most rigid among the portions.

The distal portion 2a includes a tapered portion 7 and a tip end portion 8. The tapered portion 7 is connected to a distal end portion of the center portion 2c and has a circular cross section whose diameter decreases toward the tip end portion 8. The tip end portion 8 is connected to the tapered portion 7.

The tip end portion 8 has a flat shape and has a long rectangular cross section when taken in a direction perpendicular to a longitudinal direction of the core shaft 2.

The rigidity of the distal portion 2a thus gradually decreases from the proximal side to the distal side and thus the flexibility of the distal portion 2a increases toward the distal side. Particularly, the tip end portion 8 having a flat shape is highly flexible. The distal portion 2a may entirely be formed in a flat shape or a tapered shape.

The proximal portion 2b is cylindrical and has a maximum diameter that is larger than the maximum diameters of the distal portion 2a, the center portion 2c, the first joint portion 2d₁, and the second joint portion 2d₂. A joint portion for connecting an extension guidewire may be attached to a proximal end of the proximal portion 2b.

The center portion 2c has a tapered shape such that the diameter of the center portion 2c decreases from the proximal end portion side to the distal end portion side. The center portion 2c has a circular cross section when taken in a direction perpendicular to the longitudinal direction.

The diameter of the distal end portion of the center portion 2c coincides with the diameter of the proximal end portion of the distal portion 2a, and the diameter of the proximal end portion of the center portion 2c coincides with the diameter of the distal end portion of the joint portion 2d (first joint portion 2d₁). The diameter of the proximal end portion of the center portion 2c preferably ranges from 0.1 to 0.3 mm, and the diameter of the distal end portion of the center portion 2c preferably ranges from 0.05 to 0.15 mm. The length of the center portion 2c preferably ranges from 100 to 300 mm. These dimensions are preferable because, when the center portion 2c having these dimensions is covered with the hollow resin body 4, a tube region X can have a desirable gradually-changing structure.

The cylindrical first joint portion 2d₁, which is connected to the center portion 2c, and the tapered second joint portion 2d₂, which is connected to the proximal portion 2b, constitute the joint portion 2d that is disposed between the center portion 2c and the proximal portion 2b.

The core shaft 2 may be made of a material such as stainless steels, superelastic alloys including a nickel-titanium (Ni-Ti) alloy, a piano wire, and a tungsten wire. Examples of stainless steels include martensitic stainless steels, ferritic stainless steels, austenitic stainless steels, austenitic-ferritic duplex stainless steels, and precipitation hardening stainless steels. Austenitic stainless steels are the most preferable among these steels and particularly, austenitic stainless steels of Japanese Industrial Standards (JIS) No. SUS304, SUS316, and SUS316L are preferable.

The distal hollow coil body 3a is a tubular body having a through hole and is formed by helically winding a single element wire 3a₁. The distal hollow coil body 3a may be formed by helically winding multiple element wires.

The element wire 3a₁ constituting the distal hollow coil body 3a is preferably made of a stainless steel, such as a martensitic stainless steel, a ferritic stainless steel, an austenitic stainless steel, an austenitic-ferritic duplex stainless steel, or a precipitation hardening stainless steel, a superelastic alloy including a Ni-Ti alloy, or tungsten.

A hollow stranded body 5 is disposed inside the distal hollow coil body 3a.

The hollow stranded body 5 is a tubular body having a through hole therein and is formed by helically twisting multiple element wires 5a.

The hollow stranded body 5 covers the distal portion 2a when the distal portion 2a is housed in the inside of the hollow stranded body 5.

The distal hollow coil body 3a covers the hollow stranded body 5 and the distal portion 2a when the hollow stranded body 5 and the distal portion 2a are housed in the distal hollow coil body 3a. The distal hollow coil body 3a, the hollow stranded body 5, and the distal portion 2a constitute a distal region Y.

An element wire 5a constituting the hollow stranded body 5 is preferably made of the same material as the element wire 3a₁ constituting the distal hollow coil body 3a.

The tip end portion 8 of the distal portion 2a, the distal end portion of the distal hollow coil body 3a, and the distal end portion of the hollow stranded body 5 are fixed by a tip member 6. The proximal end portions of the distal portion 2a, the distal hollow coil body 3a, and the hollow stranded body 5 are fixed by a middle first soldered portion 9.

Examples of materials of the tip member 6 and the middle first soldered portion 9 include an aluminum alloy, silver, gold, zinc, a tin-lead (Sn-Pb) alloy, a tin-gold (Sn-Au) alloy, a lead-silver (Pb-Ag) alloy, and a tin-silver (Sn-Ag) alloy. Among these materials, gold, a Sn-Au alloy, and a Sn-Ag alloy are particularly favorable because the use of these material increases the strength of the soldered portion.

The hollow resin bodies 4 illustrated in Figs. 1 and 2A are tubular bodies having approximately the same outer diameter, inner diameter, and wall thickness, and each of the tubular bodies has a through hole therein. The hollow resin bodies 4 illustrated in Figs. 1 and 2A may each have a substantially uniform outer diameter, a substantially uniform inner diameter, and a substantially uniform wall thickness. Each hollow resin body 4 covers a corresponding one of the center portion 2c when the center portion 2c is housed in the hollow resin body 4. The hollow resin body 4 and the center portion 2c constitute a tube region X.

The wall thickness of the hollow resin body 4 preferably ranges from 0.05 to 0.1 mm. The length of the hollow resin body 4 is preferably the same as the length of the center portion 2c, and thus ranges from 100 to 300 mm. These dimensions are preferable because the tube region X can have a desirable gradually-changing structure when the center portion 2c is covered with the hollow resin body 4 having these dimensions.

Preferable examples of materials of the hollow resin body 4 include a polyarylene sulfide, polyalkyd, polystyrene, polyester, polyamide, polyaramide, polyamido-imide, polyarylate, polyarylsulfone, polyether sulfone, polyimide, polyetherimide, polytetrafluoroethylene, polyether ketone, polyether ether ketone, polyether ketone ketone, polybenzoxazole, polyoxadiazole, polybenzothiazinophenothiazine, polybenzothiazole, polypyrazinoquinoxaline, polypyromellitimide, polyquinoxaline, polybenzimidazole, polyoxindole, polyoxoisoindoline, polydioxoisoindoline, polytriazine, polypyridazine, polypiperazine, polypyridine, polypiperidine, polytriazole, polypyrazole, polycarborane, polyoxabicyclononane, polydibenzofuran, polyphthalide, polyacetal, polyanhydride, polyvinyl ether, polyvinyl thioether, polyvinyl alcohol, polyvinyl ketone, polyvinyl halide, polyvinyl nitrile, polyvinyl ester, polysulfonate, polysulfide, polysulfonamide, polyurea, polyphosphazine, polysilazane, polyolefin, polysiloxane, and a combination of materials including at least one of these thermoplastic polymers. Among these, a polyimide is particularly preferable.

The hollow resin body 4, which is a tubular body, has a higher rigidity and is less easily bent than the distal hollow coil body 3a, which is formed by helically winding a single element wire. The rigidity in this specification means a deformability (bendability) of a component when an external force is applied to the component in a direction perpendicular to the longitudinal direction of the guidewire.

The proximal hollow coil body 3b is a tubular body having a through hole therein and is formed by helically winding a single element wire 3b₁. The proximal hollow coil body 3b may be formed by helically winding multiple element wires.

The element wire constituting the proximal hollow coil body 3b is preferably made of the same material as the element wire 3a₁ constituting the distal hollow coil body 3a.

When the first joint portion 2d₁ is housed in the inside of the proximal hollow coil body 3b, the proximal hollow coil body 3b covers a part of the first joint portion 2d₁. In this manner, the hollow resin body 4 is sandwiched between and fixed by the distal hollow coil body 3a and the proximal hollow coil body 3b. Here, the proximal hollow coil body 3b may cover a part or the entirety of the first joint portion 2d₁. Although the proximal hollow coil body 3b may be omitted, it is preferable, in this case, that the first joint portion 2d₁ includes a contact portion to which the proximal end portion of the hollow resin body 4 is to be fixed.

The distal end portion of the first joint portion 2d₁ and the distal end portion of the proximal hollow coil body 3b are fixed to each other by a middle second soldered portion 10. A center portion of the first joint portion 2d₁ and the proximal end portion of the proximal hollow coil body 3b are fixed to each other by a proximal soldered portion 11.

It is preferable that the middle second soldered portion 10 and the proximal soldered portion 11 are made of the same material as the tip member 6 and the middle first soldered portion 9.

The outer diameter of the distal hollow coil body 3a, the outer diameter of the hollow resin body 4, and the outer diameter of the proximal hollow coil body 3b are approximately the same. For this reason, the guidewire 1 can smoothly pass through the inside of a blood vessel or a lesion.

The guidewire 1 according to the embodiment can be manufactured in the following manner. For example, a metallic shaft is subjected to a tapering process so as to have a predetermined shape, so that a core shaft 2 is formed. The core shaft 2 thus formed is inserted into the through holes of the proximal hollow coil body 3b, the hollow resin body 4, the hollow stranded body 5, and the distal hollow coil body 3a, and then predetermined portions of corresponding components are soldered. Examples of a tapering process include a machining process including centering and grinding, a swaging process, and a drawing process.

Operations and effects of the guidewire 1 according to the present invention will be described below.
(1) In the guidewire 1 according to the embodiment, the hollow coil body 3 covers a distal portion 2a having a smaller diameter, while the hollow resin body 4 having a higher rigidity than the hollow coil body 3 covers the tapered center portion 2c. For this reason, the rigidity of the distal-side portion 1a of the guidewire 1 gradually decreases from the proximal end portion side to the distal end portion side of the tube region X, and further to the distal region Y. In this manner, the flexibility of the distal-side portion 1a of the guidewire 1 gradually increases toward the tip end, and thus lumens such as a blood vessel are less likely to be damaged.
(2) Since the rigidity of the tube region X gradually decreases from the proximal end portion side to the distal end portion side, the guidewire 1 is capable of following a curve of a blood vessel even when the tube region X arrives at the curved portion. Moreover, since the center portion 2c is covered by the hollow resin body 4 having a higher rigidity than the hollow coil body 3, the tube region X maintains such a degree of rigidity that the guidewire 1 does not reduce its device delivering efficiency. Thus, the guidewire 1 is less likely to meander while delivering a device, and is excellent in terms of device delivering efficiency.
(3) In the case where the hollow resin body 4 is made of a polyimide, the above effect (2) can be exerted more favorably.
(4) Since the hollow stranded body 5 covers the distal portion 2a, torque applied to a proximal-side portion 1b of the guidewire 1 can be efficiently transmitted to the tip of the distal-side portion 1a. The guidewire 1 thus has an excellent ability to transmit torque.

### Second Embodiment

A guidewire according to a second embodiment has the same configuration as the guidewire 1 according to the first embodiment except that a first end portion of a hollow resin body 4 on the distal end portion side has a smaller wall thickness than a second end portion of the hollow resin body on the proximal end portion side. Components that are the same as those of the guidewire 1 according to the first embodiment will not be described.

The configuration of the hollow resin body 4 is specifically described now. The hollow resin body 4 of the guidewire according to the embodiment has a substantially uniform outer diameter and is a tubular body having a through hole inside thereof. The wall thickness of the hollow resin body 4 gradually decreases from the second end portion side to the first end portion side. Accordingly, the hollow resin body 4 has an inner diameter that is large on the first end portion side and small on the second end portion side, and the through hole of the hollow resin body 4 has a tapered shape.

The guidewire according to the second embodiment can exert the following operation and effect (5) in addition to the operations and effects (1) to (4) associated with the guidewire 1 according to the first embodiment.
(5) In addition to the center portion 2c being formed in a tapered shape and being gradually made more flexible toward the distal region Y, the first end portion of the hollow resin body 4 on the distal end portion side has a smaller wall thickness than the second end portion of the hollow resin body 4 on the proximal end portion side. The flexibility of the distal-side portion of the guidewire thus increases gradually and gently. With this configuration, the guidewire according to the embodiment is capable of following a curve of a blood vessel and is excellent in terms of device delivering efficiency.

### Third Embodiment

A guidewire according to a third embodiment has the same configuration as the guidewire according to the second embodiment except that multiple grooves are formed in a hollow resin body in a direction perpendicular to the longitudinal direction of the hollow resin body, and the number of grooves formed on the first end portion side located on the distal end portion side is larger than the number of grooves formed on the second end portion side located on the proximal end portion side. Components that are the same as those of the guidewire according to the second embodiment will not be described.

Fig. 2B schematically illustrates a hollow resin body 20 of the guidewire according to the third embodiment when viewed in perspective.

The hollow resin body 20 of the guidewire according to the embodiment illustrated in Fig. 2B has a substantially uniform outer diameter, a substantially uniform inner diameter, and a substantially uniform wall thickness, and is a tubular body having a through hole inside thereof.

Multiple grooves 21 are formed in the outer circumference of the hollow resin body 20 in a direction perpendicular to the longitudinal direction of the hollow resin body 20.

The multiple grooves 21 are arranged side by side at predetermined intervals in the longitudinal direction of the hollow resin body 20. A first end portion 22 located on the distal side has more grooves 21 than a second end portion 23 located on the proximal end portion side. Thus, the first end portion 22 has a lower rigidity and is more flexible than the second end portion 23.

The length of each groove 21 preferably ranges from approximately 1/5 to approximately 1/2 the length of the outer circumference of the hollow resin body 20. This is because, with this configuration, the first end portion 22 is made more flexible than the second end portion 23 while maintaining a certain degree of strength.

The guidewire according to the embodiment can exert the following operation and effect (6) in addition to the operations and effects (1) to (5) associated with the guidewires according to the first and second embodiments.
(6) Since the density at which grooves are formed in the first end portion is different from that at which grooves are formed in the second end portion, the first end portion located on the distal side has a lower rigidity and is more flexible than the second end portion located on the proximal end portion side. Consequently, the tube region is capable of following a curve of a blood vessel more easily, and the guidewire according to the embodiment thus has a further excellent device delivering efficiency.

### Fourth Embodiment

A guidewire according to a fourth embodiment has the same configuration as the guidewire according to the second embodiment except the following points. The guidewire according to the fourth embodiment has a helical groove that is helically cut in a hollow resin body around the longitudinal axis of the hollow resin body, and a part of a helical groove formed on the first end portion side or the distal portion side is cut at a pitch that is narrower than a pitch at which a part of the helical groove on the second end portion side or the proximal portion side is cut. Components that are the same as those of the guidewire according to the second embodiment will not be described.

Fig. 2C schematically illustrates a hollow resin body 30 of the guidewire according to the fourth embodiment when viewed in perspective.

The hollow resin body 30 of the guidewire according to the embodiment illustrated in Fig. 2C has a substantially uniform outer diameter, a substantially uniform inner diameter, and a substantially uniform wall thickness, and is a tubular body having a through hole inside thereof.

A continuous helical groove 31, which is helically cut around the longitudinal axis of the hollow resin body 30, is formed in the outer circumference of the hollow resin body 30.

A pitch L1, at which a part of the helical groove 31 on the first end portion 32 side is formed, is narrower than a pitch L2, at which a part of the helical groove 31 on the second end portion 33 side is formed. The pitch of the helical groove 31 gradually increases from the pitch L1 to the pitch L2 as the helical groove 31 moves from the first end portion 32 side toward the second end portion 33 side. Consequently, the first end portion 32 has a lower rigidity and thus is made more flexible than the second end portion 33. Like the helical groove 31 in this embodiment, a single continuous helical groove may be simply formed, or multiple continuous helical grooves may be formed, instead.

The guidewire according to the embodiment can exert the following operation and effect (7) in addition to substantially the same operations and effects (1) to (5) associated with the guidewires according to the first and second embodiments and substantially the same operation and effect (6) associated with the guidewire according to the third embodiment.
(7) The guidewire according to the embodiment is excellent in terms of passing through a lesion because a distal-side portion of the guidewire, which has a helical groove in the outer circumference, can screw into the lesion like a screw.

### Modifications

In the guidewire according to each embodiment of the present invention, the center portion only has to have a tapered shape such that the diameter of the center portion decreases from the proximal end portion side toward the distal end portion side. The cross section of the center portion may have a shape including an oval, an ellipse, an irregular circle defined by an arc and a straight line, a rectangle, and a regular polygon having n angles (where n is a positive integer not smaller than 3), in addition to the above described circular shape. Accordingly, the "diameter of the center portion" refers to a diameter of a cycle circumscribing or enclosing a cross-section of the center portion.

In the guidewire according to each embodiment of the present invention, the hollow stranded body may or may not be disposed inside the hollow coil body. If the hollow stranded body is not disposed inside the hollow coil body, the distal-side portion of the guidewire can have a higher flexibility.

In the guidewire according to each embodiment of the invention, the first end portion of the hollow resin body located on the distal side may have a wall thickness that is larger than a wall thickness of the second end portion of the hollow resin body on the proximal end portion side. In this configuration, the rigidity of the tube region on the distal end portion side can increase, and this configuration is thus preferable if a higher device delivering efficiency is to be achieved.

In the case where the guidewire according to each embodiment of the present invention has multiple grooves formed in the hollow resin body in a direction perpendicular to the longitudinal direction of the hollow resin body, the number of grooves formed on the first end portion side and the number of grooves formed on the second end portion side may substantially be the same.

In the case where the guidewire according to each embodiment of the present invention has a helical groove that is helically cut in the hollow resin body around the longitudinal axis of the hollow resin body, a part of the helical groove on the first end portion side may be formed at substantially the same pitch at which a part of the helical groove on the second end portion side is formed.

In the guidewire according to each embodiment of the present invention, a hollow resin body may be constituted by short hollow resin units that are jointed to each other in the longitudinal direction. In this case, it is preferable that each unit be made of the same material as the hollow resin body according to the first embodiment. The units may be made of the same material or different materials. In the case where the units are made of different materials, it is preferable that a resin of which a distal portion side (first end portion side) unit is made have a smaller rigidity than a resin of which a proximal end portion side (second end portion side) unit is made so that the distal portion side of the hollow resin body is made more flexible.

In the guidewire according to each embodiment of the present invention, the outer surface of the guidewire may be covered with a hydrophilic material. By covering the outer surface with a hydrophilic material, a guidewire can slide into a guiding catheter, a tube, or a body organ with a low friction, and thus is capable of moving smoothly.

Examples of hydrophilic materials include a cellulose-based macromolecular substance, a polyethylene oxide-based macromolecular substance, a maleic anhydride-based macromolecular substance (a maleic anhydride copolymer such as a methyl vinyl ether-maleic anhydride copolymer), an acrylamide-based macromolecular substance (for example, polyacrylamide or a block copolymer of polyglycidylmethacrylate-dimethylacrylamide), water-soluble nylon, polyvinyl alcohol, polyvinyl pyrrolidone, and hyaluronan. Among these, hyaluronan is most preferable.

In the guidewire according to each embodiment of the invention, when multiple grooves are formed in the hollow resin body in a direction perpendicular to the longitudinal direction of the hollow resin body and if the number of grooves formed on the first end portion side or the distal end portion side is larger than the number of grooves formed on the second end portion side or the proximal end portion side, it is preferable, as described in the third embodiment, that the first end portion of the hollow resin body on the distal end portion side has a smaller wall thickness than the second end portion on the proximal end portion side. The entire wall thickness of the hollow resin body, however, may be almost uniform, as described in the first embodiment. In the guidewire according to each embodiment of the invention, when a helical groove is helically cut in the hollow resin body around the longitudinal axis of the hollow resin body and if a pitch at which a part of the helical groove on the first end portion side or the distal end portion side is formed is narrower than a pitch at which a part of the helical groove on the second end portion side or the proximal end portion side is formed, it is preferable, as described in the fourth embodiment, that the first end portion of the hollow resin body on the distal end portion side has a smaller wall thickness than the second end portion on the proximal end portion side. The entire wall thickness of the hollow resin body, however, may be almost uniform, as described in the first embodiment.

## Claims

1. A guidewire (1) comprising:
a core shaft (2) including a distal portion (2a) and a center portion (2c) that is adjacent to the distal portion (2a);
a hollow coil body (3, 3a) covering the distal portion (2a); and
a hollow resin body (4, 20, 30) covering the center portion (2c)
**characterized in that**
the center portion (2c) has a tapered shape such that the diameter of the center portion (2c) decreases from a proximal end portion side of the center portion (2c) toward a distal end portion side of the center portion (2c), and
a rigidity of the hollow resin body (4, 20, 30) is higher than a rigidity of the hollow coil body (3, 3a).

2. The guidewire (1) according to claim 1,
wherein the core shaft further includes a joint portion (2d) that is adjacent to the center portion (2c) and located on a side that is opposite that of the distal portion (2a),
wherein the joint portion (2d) is covered with a second hollow coil body (3, 3b), and
wherein the hollow resin body (4, 20, 30) is sandwiched between the hollow coil body (3, 3a) and the second hollow coil body (3, 3b).

3. The guidewire (1) according to claim 1 or 2,
wherein a first end portion (22, 32) of the hollow resin body (4, 20, 30) located on a distal portion side has a wall thickness that is smaller than a wall thickness of a second end portion (23, 33) of the hollow resin body (4, 20, 30) located on a joint portion side.

4. The guidewire (1) according to one of claims 1 to 3,
wherein a plurality of grooves (21) are formed in the hollow resin body (20) in a direction perpendicular to a longitudinal direction of the hollow resin body (20), and
wherein the number of grooves (21) formed on a first end portion side (22) of the hollow resin body (20) is larger than the number of grooves (21) formed on a second end portion side (23) of the hollow resin body (20).

5. The guidewire (1) according to one of claims 1 to 3,
wherein the hollow resin body (30) has a helical groove (31) that is helically cut around an longitudinal axis of the hollow resin body (30), and
wherein a pitch at which a part of the helical groove (31) on a first end portion side (32) of the hollow resin body (30) is formed is narrower than a pitch at which a part of the helical groove (31) on a second end portion side (33) of the hollow resin body (30) is formed.

6. The guidewire according to one of claims 1 to 5,
wherein a hollow stranded body (5) obtained by twisting a plurality of element wires together is disposed inside the hollow coil body (3, 3a), and
wherein the hollow stranded body (5) covers the distal portion (2a).
